# EUROPEAN PATENT APPLICATION

(11) **EP 3 815 639 A1**
(43) Date of publication of application: **05.05.2021**
(21) Application number: 19206079.6
(22) Date of filing: 30.10.2019
(51) Int. Cl.: A61B 17/88

(54) **DISPENSING MECHANISM AND METHOD OF DISPENSING MATERIAL USING A DISPENSING MECHANISM**

(71) Applicant: Sulzer Mixpac AG, 9469 Haag (Rheintal) (CH)
(72) Inventor: MOSER, Josef, 5444 Künten (CH); MATHYS, Beat, CH-5630 (CH); VEID, Martin, 6353 Weggis (CH); KUGLER, Stefan, 8047 Zürich (CH)
(74) Representative: Manitz Finsterwald Patent- und Rechtsanwaltspartnerschaft mbB

(57) **Abstract**

The present invention relates to a dispensing mechanism for dispensing material from a cartridge, the dispensing mechanism comprising a sleeve, a cartridge plunger, and an engagement part at said cartridge plunger for rotating at least one of the cartridge plunger and said sleeve, wherein first and second holding members are present between the cartridge plunger and said sleeve, with the first and second holding members being configured to hold the cartridge plunger at the sleeve when a torque below a pre-defined threshold is applied at the cartridge plunger. The invention further relates to a method of dispensing material using a dispensing mechanism.

## Description

The present invention relates to a dispensing mechanism for dispensing material from a cartridge, the dispensing mechanism comprising a sleeve, a cartridge plunger, and an engagement part at said cartridge plunger for rotating at least one of the cartridge plunger and said sleeve, wherein first and second holding members are present between the cartridge plunger and said sleeve, with the first and second holding members being configured to hold the cartridge plunger at the sleeve when a torque below a pre-defined threshold is applied at the cartridge plunger. The invention further relates to a method of dispensing material using a dispensing mechanism.

Mixing and dispensing systems are known to mix two-component materials, such as the ones used in joint replacement surgery. On mixing two-component material, such as cement, e.g. PMMA (poly-methyl methacrylate) used e.g. in joint replacement surgery, such as hip replacement surgery, cracks in the cement mantle may facilitate a mechanical loosening of the prosthesis.

Moreover, the cement is to be used in a sterile environment and may be rather harmful to the user of the mixing and dispensing system which is why the possible contact of the user with the cement prepared in the mixing and dispensing system is not desirable.

In order to dispense mixed materials from the cartridge multi-use material dispensers are known, such as the ones present in the form of a dispensing gun. However, multi-use tools have to be sterilized and calibrated for multiple uses within a sterile environment.

For this reason it is an object of the invention to provide dispensing mechanism that can be used efficiently in a sterile environment with which a dispensing of materials present in the cartridge can be carried out accurately and without excessive force having to be used without having to repeatedly sterilize and/or calibrate said dispensing mechanism. Further it is desirable to provide a dispensing mechanism with which the contact a user of the mixing and dispensing system may have with the two-component material to be mixed can be minimized. It is a further object of the present invention to provide a dispensing mechanism which can be produced in an as facile as possible manner.

This object is satisfied by a mixing and dispensing system having the features of claim 1.

Such a dispensing mechanism for dispensing material from a cartridge comprises a sleeve having a first thread arranged at an outer surface thereof for connecting the sleeve to an end of the cartridge; a cartridge plunger that can be arranged partly within said sleeve, the cartridge plunger being moveable with and relative to said sleeve, and the cartridge plunger having a second thread arranged at an outer surface thereof that can interact with an inner thread present at an inner surface of the sleeve; and an engagement part at said cartridge plunger for rotating at least one of the cartridge plunger and said sleeve; wherein first and second holding members are present between the cartridge plunger and said sleeve, with the first and second holding members being configured to hold the cartridge plunger at the sleeve when a torque below a pre-defined threshold is applied at the cartridge plunger.

By providing two different threaded portions a multi-speed dispensing mechanism can be made available via which a fast attachment to a cartridge can be brought about while simultaneously providing a dispensing mechanism via which one can accurately dispense materials from a cartridge. The first and second holding members cooperate in the manner of a torque wrench so that the sleeve can be held relative to the cartridge plunger.

Such a single use dispensing mechanism can for example be produced cost effectively and swiftly in an injection molding process without the need to repeatedly sterilize and/or calibrate the dispensing mechanism as this can be produced in a sterile environment and be sterile from the onset.

Moreover, since the dispensing mechanism comprises an engagement part which can be held and engaged by a user the contact a user of the mixing and dispensing system may have with the two-component material to be mixed can be minimized.

Furthermore, such a design permits a spindle drive like dispensing mechanism to be formed which is less exhausting to use in comparison to a power tool can be made available by means of the dispensing mechanism discussed herein.

On entraining the sleeve into the cartridge via the cartridge plunger one can apply a reduced force at the cartridge plunger, whereas once the sleeve experiences an increased resistance from the cartridge on reaching its final position the pre-defined threshold can be achieved in order to set free the cartridge plunger such that this can be entrained relative to the sleeve.

The first and second holding members may be configured to release the cartridge plunger from the sleeve once a torque above a pre-defined threshold is applied at the cartridge plunger. In this way one can ensure that the cartridge plunger can be entrained relative to the sleeve.

The first holding member may be formed at an outer surface of the cartridge plunger and comprises a plurality of teeth. In this way the first member can be arranged as close as possible to the sleeve. The use of teeth in a torque wrench like application ensures an entraining at comparatively low torque values and a slipping over the teeth in a manner known per se.

The second holding member may be formed at the inner surface of the sleeve and comprises a plurality of cams. The use of teeth in a torque wrench like application has been tried and tested and is simple to produce.

The plurality of teeth and the plurality of cams are formed complementary to one another. In this way the teeth and the cams can be matched to one another to ensure a stable operation of the first and second holding members.

The plurality of teeth is configured to slip over the plurality of cams when a torque applied at said cartridge plunger exceeds the pre-defined threshold. In this way the first and second holding members can be configured in the manner of a slipper like torque wrench.

The first and second threads may have different pitches. In this way one can ensure that two different speeds of entraining the dispensing mechanism relative to the cartridge can be achieved.

The second thread may be finer than the first thread, i.e. the second thread may have a lower thread pitch than the first thread. The spindle drive is thus designed in a way that allows a fast connection of the spindle drive to the cartridge at the beginning of rotation and changes over to a lower pitch transmitting higher forces when needed.

The dispensing mechanism may further comprise a cartridge, optionally filed with material, wherein the first thread is configured to engage an inner surface of a cartridge wall of the cartridge. Such a dispensing mechanism can be provided in a sterile packaging with materials already pre-stored in the cartridge which reduces the risk of contamination in a sterile environment.

The sleeve may be configured to release a piston present in said cartridge from a storage position of said piston. In this way the piston can act as a seal to preserve the materials pre-stored in the cartridge ensuring an increased storage life of such pre-packed sterile dispensing mechanisms.

The cartridge plunger may be configured to move the piston present in said cartridge to and fro along a longitudinal axis of said cartridge once the sleeve has released the piston from the storage position of said piston. Thus the cartridge plunger can directly interact with the material(s) stored in the cartridge for the purpose of dispensing.

In this connection it should be noted that also single component materials can be stored in the cartridge and/or dispensed with the dispensing mechanism.

The cartridge plunger may comprise a passage configured for receiving a mixing shaft of a mixer arranged within said cartridge. Thereby the two-component material can be stored in the cartridge and mixed shortly before its application without the user of the dispensing mechanism running the risk of coming into contact with the material to be dispensed.

The sleeve may comprise an abutment at the outer surface thereof for abutting against an end of the cartridge. Such an abutment defines the maximum amount the sleeve may travel relative to the cartridge increasing the degree of accuracy of use of the dispensing mechanism.

The abutment may be arranged at the same axial height of the sleeve as the second holding member. In this way the sleeve may be configured as compact as possible reducing the size of the components.

According to a further aspect the present invention relates to a method of dispensing material using a dispensing mechanism, the method comprising the steps of:
- rotating an engagement part of said cartridge plunger while connecting the sleeve to the cartridge; and
- further rotating said engagement part to rotate only said cartridge plunger once a torque has been applied at said cartridge plunger that is above said pre-defined threshold.

In this way the dispensing mechanism discussed in the foregoing can be advantageously put to use.

Further embodiments of the invention are described in the following description of the Figures. The invention will be explained in the following in detail by means of embodiments and with reference to the drawing in which is shown:
- Fig. 1a: a side view of components of a mixing and dispensing system;
- Fig. 1b: a sectional view taken along the sectional line B:B of Fig. 1a of the mixing and dispensing system;
- Fig. 2a: an enlarged view of the activation element of Fig. 1a;
- Fig. 2b: an enlarged view of the section through the activation element of Fig. 1b;
- Fig. 3a: an enlarged sectional view of a handle of the mixing and dispensing system shown in Fig. 1b;
- Fig. 3b: an enlarged side view of the handle of Fig. 3a;
- Fig. 3c: an exploded view of the handle of Fig. 3b partly removed from a mixing shaft;
- Fig. 4a: a sectional view similar to the one of Fig. 1b with a threaded dispenser and a dispensing outlet arranged at a cartridge of the mixing and dispensing system;
- Fig. 4b: a part sectional part schematic view of the threaded dispenser of Fig. 4a;
- Fig. 4c: an enlarged view of the section shown in Fig. 4a in the region of a piston driving end of the cartridge;
- Fig. 4d: a part sectional part schematic view of first and second threads of the threaded dispenser of Fig. 4a;
- Fig. 4e: an enlarged partial view of part of the threaded dispenser of Fig. 4a;
- Fig. 4f: a sectional view taken along the section A:A of Fig. 4e; and
- Fig. 4g: an enlarged view of part of the section of Fig. 4f.

In the following the same reference numerals will be used for parts having the same or equivalent function. Any statements made having regard to the direction of a component are made relative to the position shown in the drawing and can naturally vary in the actual position of application.

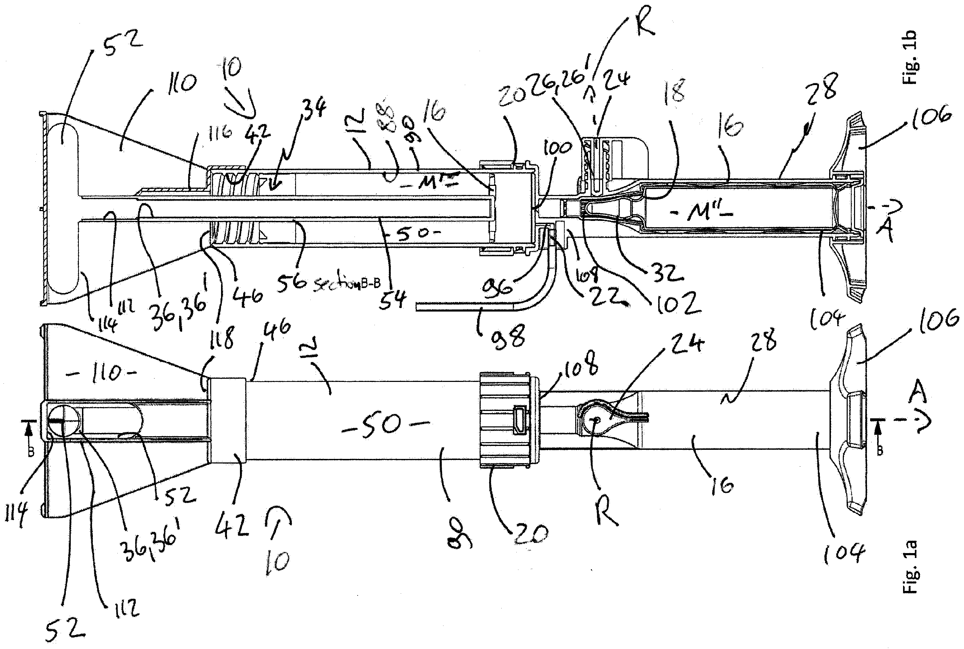

Fig. 1a shows a side view of components of a mixing and dispensing system 10 for mixing a two-component material M', M", such as a bone cement. Fig. 1b shows a sectional view taken along the sectional line B:B of Fig. 1a of the mixing and dispensing system 10.

The mixing and dispensing system 10 comprises a cartridge 12 for storage of a first component M' of the two-component material M', M", the cartridge 12 comprising a mixer 14 arranged therein.

An ampoule container 16 for receiving an ampoule 18 comprising a second component M" of the two-component material M', M" is connected to the cartridge 12 via a first interface 20. The first interface 20 is configured to releasably connect and disconnect the cartridge 12 with and from said ampoule container 16. A longitudinal axis A extends between said cartridge 12 and said ampoule container 16 and through said first interface 20.

The ampoule container 16 further comprises an activation element 24. On moving said activation element 24 from a storage position into an activated position, a part 26 of said activation element 24 moves perpendicular to said longitudinal axis A such that the part 26 contacts a top 32 of said ampoule 18 for opening said ampoule 18 when said activation element 24 is displaced from the storage position into the activated position. Thereby the second component M" stored in said ampoule 18 can be released from said ampoule 18 into said cartridge 12 to come into contact with said first component M' stored in said cartridge 12 for mixing the two-component material M', M" in said cartridge 12 with said mixer 14.

The activation element 24 is arranged partly at an outer wall 28 of the ampoule container 16. More specifically a rotatable switch 30, such as a half turn coupling 30', of the activation element 24 is arranged at the outer wall 28. The activation element 24 further comprises an activation plunger 26' arranged within said ampoule container 16. The activation plunger 26' is configured to contact the top 32 of said ampoule 18 for opening said ampoule 18 when said activation element 24 is displaced from the storage position into the activated position.

In this connection also a quarter turn coupling or the like would be feasible to activate the activation plunger 26'.

In order to displace the activation plunger 26' from the storage position into the activated position, the rotatable switch 30 is rotated about an axis of rotation R to produce an axial movement of said activation plunger 26' along said axis of rotation R of said rotatable switch 30.

A vacuum port 22 is also arranged at the ampoule container 16 for providing a vacuum within the mixing and dispensing system 10. The vacuum port 22 is arranged remote from the activation element 24 in such a way that it does not come into contact with the rotatable switch 30 on rotating the rotatable switch 30 about the axis of rotation R.

On mixing two-component material M', M", such as cement M, M", e.g. PMMA (poly-methyl methacrylate) used e.g. in joint replacement surgery, such as hip replacement surgery, cracks in the cement mantle may facilitate a mechanical loosening of the prosthesis. In order to avoid this a vacuum is applied on mixing the cement M', M" in the cartridge 12 to reduce the cement porosity of the cement such that less cracks, pores etc. are formed in the cement and particularly its mantle.

In this connection it should be noted that the cartridge 12 should be sealed off to the outside during the mixing of the two-component material M', M", such that a contamination or the like of the two-component material M', M" can be avoided.

For this purpose the vacuum port 22 is arranged at the outer wall 28 of the ampoule container 16 such that it can evacuate a chamber 50 of the cartridge 12 formed between a piston driving end 46 and an outlet end 48 of the cartridge 12.

In order to evacuate the chamber 50, a groove 96, in particular an L-shaped groove 96, leads from the vacuum port 22 to the chamber 50 through the first interface 20 to the cartridge 12. The vacuum port 22 can be connected to a source of vacuum (not shown) via a vacuum line 98 in a manner known per se.

In this connection it should be noted that a corresponding passage or groove can also be provided extending from the vacuum port 22 to the chamber 50 if the vacuum port 22 is arranged at the cartridge 12 rather than at the ampoule container 16.

It should further be noted in this connection that it is preferred to have the vacuum port 22 at the ampoule container 16 since this makes the handling of the cartridge 12 simpler after mixing and disconnecting of the ampoule container 16, since no vacuum port 22 would then be present at the cartridge 12.

The mixer 14 is also arranged within the chamber 50 between the piston 34 and the outlet end 48, with the mixer 14 being moveable relative to the piston 34 and the chamber 50 during mixing of the cement M', M". The mixer 14 can be moved radially about said longitudinal axis A and axially along said longitudinal axis A for mixing said two-component material M', M".

A piston 34 is arranged moveable to and fro between the piston driving end 46 and the outlet end 48 of the cartridge and is stored initially at the piston driving end 46.

The piston 34 is pre-positioned within the cartridge 12 such that once a force above a threshold pressure is applied on the piston 34, the piston 34 can dispense the mixed two-component material M', M" from the cartridge. Such forces can typically be selected in the range of 100 to 3000 N.

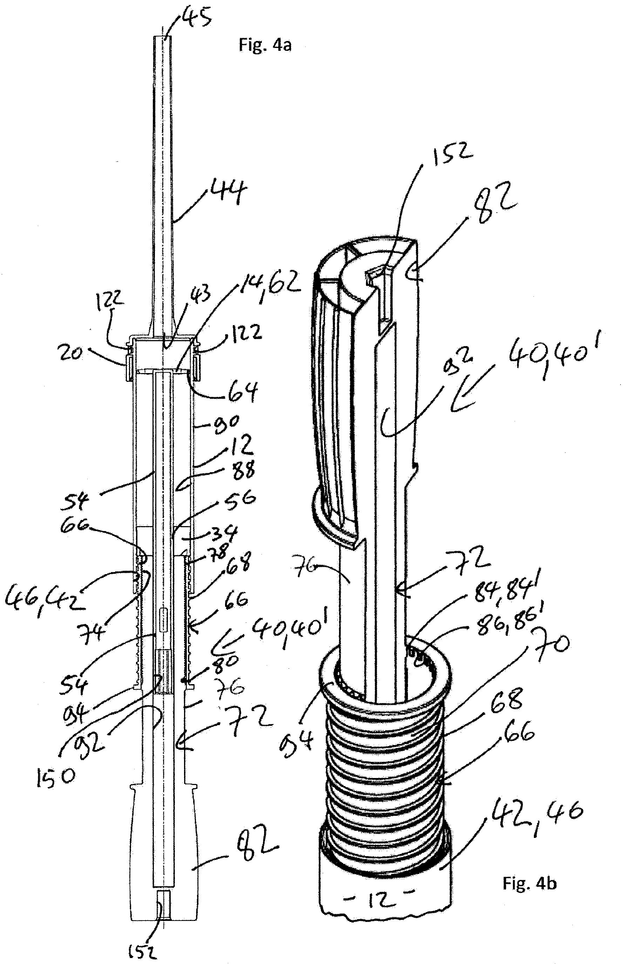

The piston 34 may be moveable via a threaded dispenser 40 (see Fig. 4a) following the release of the piston 34 from the storage position for dispensing said two-component material M', M" from said cartridge 12.

Once the ampoule 18 has been opened the second component M" stored therein is moved into the cartridge 12 via a passage 100 arranged at the ampoule container 16 for mixing in the chamber 50.

In order to avoid glass splinters (not shown) from being moved together with the second component M" into the cartridge 12, a net 102 is arranged around the top 32 of the ampoule 18.

A cap 106 can be placed onto the end 104 of the ampoule container 16 remote from the end 108 present at the first interface 20. The cap 106 can act as a stand for the ampoule container 16, such that on storing and/or activating the mixing and dispensing system 10, the mixing and dispensing system 10 can stand on a surface via this cap 106.

A part 36, more specifically a rod 36', of the mixer 14 is detachable via a connector 38 of a drive interface 38', in such a way that the threaded dispenser 40 of a dispensing mechanism 40' (see Fig. 4a) can be connected to the mixing and dispensing system 10 via a second interface 42 of the cartridge 12 at the piston driving end 46.

The mixer 14 is connected to a handle 52 via a mixing shaft 54 and the rod 36'. The mixing shaft 54 extends between the handle 52 and the mixer 14 and passes through the piston 34 via a piston passage 56 such that the mixer 14 can be moved in dependence on movements initiated via the handle 52 and indeed relative to the piston 34 and the chamber 50.

The handle 52 is detachable, in particular in a non-destructive manner, from the mixing shaft 54 via the connector 38. In this connection it should be noted that the handle 52 can also be removed without the use of tools, i.e. in a tool-free manner.

On storing the mixing and dispensing system 10, the handle 52 and part of the mixing shaft 54 are stored in a stand 110 that comprises respective grooves 112, 114 for receiving and storing the handle 52 and part of the mixing shaft 54. An end 118 of the stand is arranged adjacent to the piston driving end 46 in the storage state of the mixing and dispensing system 10. As indicated in Fig. 1b, the stand 110 may comprise a support 116 to reinforce the stand 110 and to ensure the handle 52 and thus the mixer 14 is not moved in the storage state of the mixing and dispensing system 10.

In this connection it should be noted that the precise position of the mixer 14 within the cartridge 12 is not pre-defined in the storage state of the mixing and dispensing system 10, the stand 110 should merely ensure that the mixer 14 does not move in the storage state of the mixing and dispensing system 10.

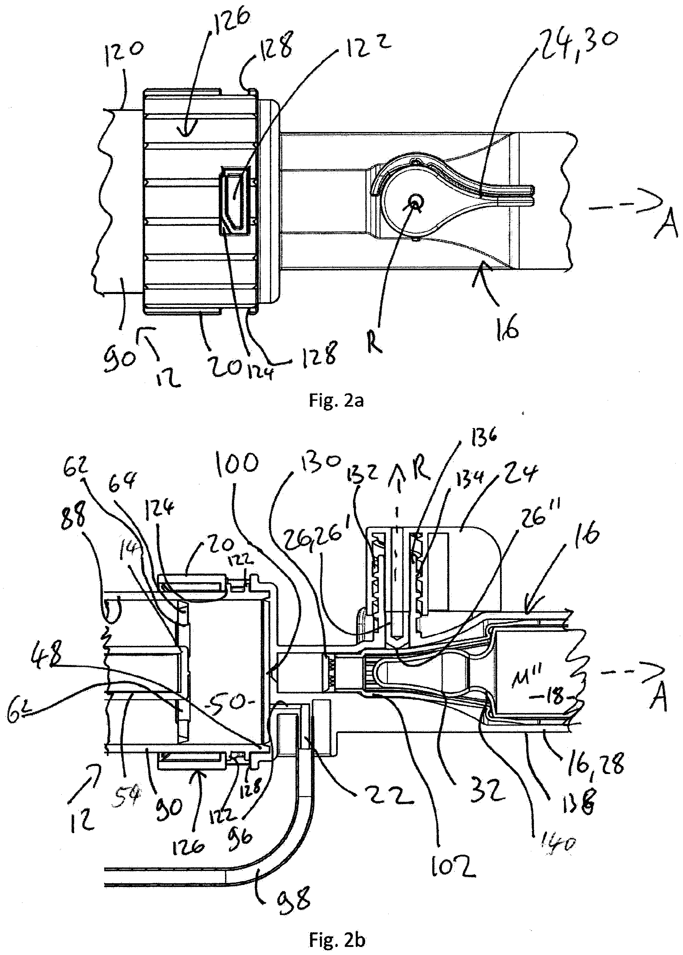

Fig. 2a shows an enlarged view of the first interface 20 and of the activation element 24 of Fig. 1a. The first interface 20 is formed by a bayonet type interface in order to releasably couple the ampoule container 16 to the cartridge 12. As indicated in Fig. 4a also a dispensing outlet 44 can be releasably coupled to the cartridge 12 via the first interface 20.

To form the first interface 20 an outer surface 120 of the cartridge 12 comprises pins 122 extending radially therefrom. The pins 122 engage matching slots 124 (see also Fig. 2b) present in a cap 126 of the first interface 20. In Fig. 2a apertures 128 of the slots 124 are visible. In this connection it should be noted that between 2 and 6, preferably between 2 and 4 pins 122 with matching slots 124 can be provided. The cap 126 in this way sits on the outlet end 48 of the cartridge 12.

A distance between the cap 126 and the activation element 24 shown is such that the switch 30 can be rotated by at most approximately 270° relative to the ampoule container 16. This means that a distance the activation plunger 26' can travel between the storage position and the activation position has to be achieved by at most a 270° degree turn of the rotatable switch 30, with the illustrated design of the rotatable switch 30.

If more turns are desired then a distance between the cap 126 and the rotatable switch 30 has to be increased, such that these components do not interfere with one another.

Additionally or alternatively, the activation element 24 could be snap fit into place and the rotation thereof can be used solely for displacing the activation plunger 26' along the axis of rotation R.

In the present instance, it has been found to be prudent to use a half turn coupling as the rotatable switch 30 as in this way one achieves a maximum distance the plunger 26' can travel in a well-defined manner through a minimum rotation of the rotatable switch 30 to achieve the opening of an ampoule 18 stored in the ampoule container 16.

Fig. 2b shows an enlarged view of the section through the first interface 20 and the activation element 24 of Fig. 1b. A membrane 130 is visible between the top 32 of the ampoule and the cap 126. This membrane 130 aids in preventing glass splinters from arriving in the cartridge 12.

As is further evident in the sectional drawing indicated in Fig. 2b, the plunger 26' is arranged at the rotatable switch 30. To bring about the rotation of the rotatable switch 30, this comprises an inner thread 132, which can rotate on an outer thread 134 of a hollow projection 136 projecting radially outwardly from an outer surface 138 of the ampoule container 16.

The plunger 26' projects from within the rotatable switch 30 through the outer wall 28 of the ampoule container 16 via the hollow projection 136, so that a tip 26" of the plunger 26' can contact the top 32 of the ampoule 18 above a neck 140 of the ampoule 18 in order to break and/or open the ampoule 18.

For the purpose of breaking and/or opening the top 32 of the ampoule, the tip 26" may have a specific shape, such as the frustoconical shape shown in Fig. 2b, or also have a conical shape or have a rounded shape.

In this connection it should be noted that the rotatable switch 30 may be integrally formed together with the plunger 26' having the tip 26" as one piece in an injection molding process.

The specific design of the mixer 14 is also shown in Fig. 2b. The mixer 14 is moveable axially along the longitudinal axis A between the piston 34 and the outlet end 48 and is rotatable about the longitudinal axis A. In order to carry out the mixing the mixer comprises several vanes 62 extending radially outward from the mixing shaft 54. At their radial outer ends the vanes 62 are connected to an outer ring 64. By way of this mixer 14 design the two-component material M', M" can be thoroughly mixed. Also other designs of mixers 14 are possible (not shown).

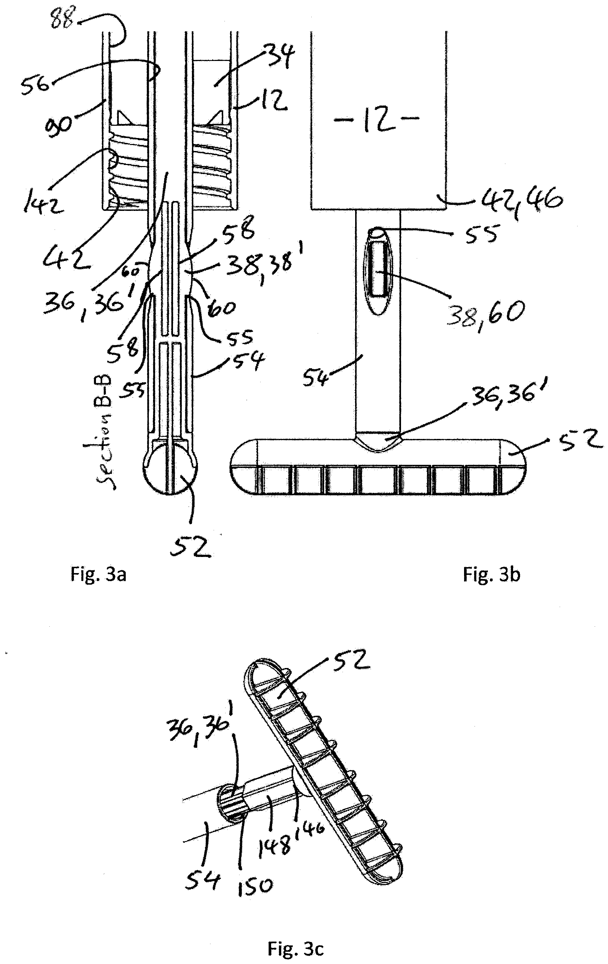

Fig. 3a shows an enlarged sectional view of the handle 52 of the mixing and dispensing system 10 shown in Fig. 1b. The mixing shaft 54 extends between the handle 52 and the mixer 14 through the piston 34 via the piston passage 56 and into the cartridge 12. The mixing shaft 54 is journaled by said piston 34 in the piston passage 56.

To connect the handle 52 to the mixing shaft 54, a rod 36' that is non-releasably connected to the handle 52 projects into said mixing shaft 54 and is thereby journaled by said mixing shaft 54. The rod 36' extends from the handle 52 beyond the connector 38 towards the mixer 14 within said mixing shaft 54.

In order to release the handle 52 from the mixing shaft 54, the connector 38 has to be arranged outside of the cartridge 12 for actuation thereof. On releasing the handle 52 two buttons 60 have to be depressed to disengage the handle 52 from the mixing shaft 54.

This is possible in the present example, since the rod 36' comprises elements 58 more flexible than the mixing shaft 54 at the connector 38. On pressing the buttons 60, the buttons 66 press the elements 58 such that they deflect inwardly so that the rod 26' of the handle can disengage from the mixing shaft 54.

This is made possible, since the elements 58 are able to deflect relative to the mixing shaft 54 and hence are more flexible than the mixing shaft 54, due to less material being provided and the elements 58 therefore being less rigid than the mixing shaft 54.

Also other methods of releasably and non-destructively releasing the handle 52 from the mixing shaft are possible, for example using threaded components (not shown).

Part of the second interface 42 is also indicated in Fig. 3a, where an inner thread 142 is present at an inner surface 80 of a cartridge wall 90 of the cartridge 12, via which second interface 42 the threaded dispenser 40 (see Fig. 4a) can be releasably coupled to the cartridge 12.

Fig. 3b shows an enlarged side view of the handle of Fig. 3a. The handle 52 is formed by a bar 144 in this example, but also other shapes are possible, such as a knob or the like. The bar 144 of the handle 52 is arranged perpendicular to the mixing shaft 54.

Fig. 3c shows an exploded view of the handle 52 of Fig. 3b partly removed from a mixing shaft 54. The connector 38 is part of a drive interface 38' for applying translational as well as rotational forces initiated via said handle 52 at the mixer 14 specifically via said rod 36' and mixing shaft 54.

For this purpose and as indicated in Fig. 3c an end 146 of the rod 36 connected to the handle 52 may comprise a hexagonal fitting 148. The hexagonal fitting 148 engages a hexagonal shaped socket 150 formed in the mixing shaft 54. In this way both translational and rotational forces can be transmitted from the handle 52 to the mixer 14 via this tight fit respectively force fit detachable connection. In this connection it should be noted that also other kinds of detachable connections are possible provided they permit a transmission of rotational and translational forces from the handle 52 to the mixer 14.

Fig. 4a shows a sectional view similar to the one of Fig. 1b with the threaded dispenser 40, i.e. a dispensing mechanism 40', arranged at the piston driving end 46 and the dispensing outlet 44 arranged at the outlet end 48 of the cartridge 12 of the mixing and dispensing system 10.

The dispensing outlet 44 is releasably connectable to said cartridge 12 via said first interface 20 on removal of said ampoule container 16 (see Figs. 1a to 2b). The dispensing outlet 44 shown has an inlet 43 at its end adjacent the first interface 20 and an outlet 45 at its opposite end, with an inner diameter of the dispensing outlet 44 tapering from the inlet 43 towards the outlet 45, such that the outlet 45 has a smaller diameter than the inlet 43. The dispensing outlet 44 thus has a frustoconical shape. In this connection it should be noted that also other shapes of dispensing outlets 44 can be provided (not shown).

The dispensing mechanism 40' comprises a sleeve 66 having a first thread 68 arranged at an outer surface 70 thereof for connecting the sleeve 66 to the piston driving end 46 of the cartridge 12. The dispensing mechanism 40' further comprises a cartridge plunger 72 that can be arranged partly within said sleeve 66, the cartridge plunger 72 being moveable with and relative to said sleeve 66, and the cartridge plunger 72 having a second thread 74 arranged at an outer surface 76 thereof. The second thread 74 can interact with an inner thread 78 present at an inner surface 80 of the sleeve 66. The cartridge plunger 72 comprises an engagement part 82 for rotating at least the cartridge plunger 72 and depending on the state of use of the threaded dispenser 40' also the sleeve 66.

Fig. 4b shows a part sectional part schematic view of the threaded dispenser 40 of Fig. 4a. First and second holding members 84, 86 are present between the cartridge plunger 72 and the sleeve 66. The first and second holding members 84, 86 are configured to hold the cartridge plunger 72 at the sleeve 66 when a torque below a pre-defined threshold is applied at the cartridge plunger 72 in a manner similar to a torque wrench. Moreover, the first and second holding members 84, 86 are configured to release the cartridge plunger 72 from the sleeve 66 once a torque above a pre-defined threshold is applied at the cartridge plunger 72.

In this connection it should be noted that the pre-defined threshold can be selected in the range of 0.2 to 1.2 Nm.

The first holding member 84 is formed at an outer surface 76 of the cartridge plunger 72 and comprises a plurality of teeth 84'. The second holding member 86 is formed at the inner surface 80 of the sleeve 66 and comprises a plurality of cams 86'. The plurality of teeth 84' and the plurality of cams 86' are formed complementary to one another.

The plurality of teeth 84' are configured to slip over the plurality of cams 86' when a torque applied at said cartridge plunger 72 exceeds the pre-defined threshold, in a manner similar to a slipper type torque wrench.

The engagement part 82 is formed as a handle in the embodiment shown. As the case may be the engagement part 82 may comprise a coupling point 152 such as a hexagonal socket for connecting the dispensing mechanism 40', i.e. the threaded dispenser 40 to a further component, such as a power tool or a hand tool for rotating the cartridge plunger 72.

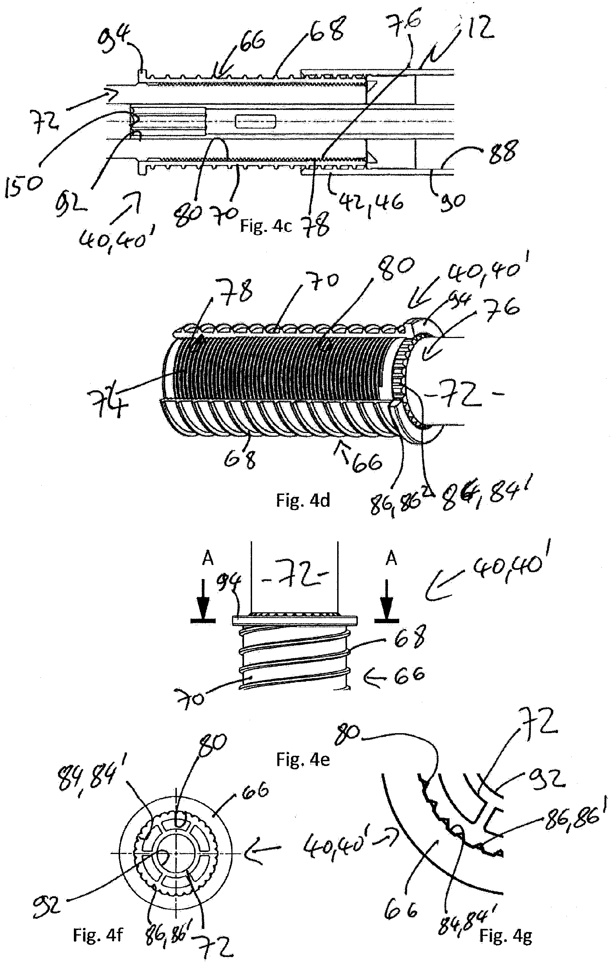

Fig. 4c shows an enlarged view of the section shown in Fig. 4a in the region of the piston driving end 46 of the cartridge 12. The first thread 68 is configured to engage the inner surface 88 of the cartridge wall 90 of the cartridge 12.

On moving the sleeve 66 into the cartridge 12 it is configured to release the piston 34 present in said cartridge 12 from a storage position of said piston 34. Once the sleeve 66 has arrived at its final position in the cartridge 12, the cartridge plunger 72 experiences a resistance so that the torque required to turn the cartridge plunger 72 further in the direction of the longitudinal axis A exceeds the pre-defined threshold, whereby the cartridge plunger 72 disengages from the sleeve 66 at the first and second holding members 84, 86 and slips out of engagement from the sleeve 66.

Then the second thread 74 starts to turn relative to the sleeve 66 in the inner thread 78 present at the inner surface 80 of the sleeve 66. This moves the cartridge plunger 72 further along the longitudinal axis A into the cartridge 12. The cartridge plunger 72 is configured to move the piston 34 present in said cartridge 12 to and fro along the longitudinal axis A of said cartridge 12 once the sleeve 66 has released the piston 34 from the storage position of said piston.

On releasing the handle 52 from the mixing shaft 54, neither the mixer 14 nor the mixing shaft 54 are removed, the threaded dispenser 40 is moved over the mixing shaft 54, which is received in a passage 92 configured for receiving the mixing shaft 54.

Fig. 4d shows a part sectional part schematic view of the first and second threads 68, 74 of the threaded dispenser 40 of Fig. 4a. The first and second threads 68, 74 have different pitches, with the second thread 74 being finer than the first thread 68, i.e. the second thread 74 has a lower thread pitch than the first thread 68.

Fig. 4e shows an enlarged partial view of part of the threaded dispenser 40 of Fig. 4a. The sleeve 66 has an abutment 94 at an end thereof remote to the end that is initially introduced into the cartridge 12. The abutment 94 comes into contact with the piston driving end 46 of the cartridge 12 once the sleeve 66 has arrived at its final position in the cartridge 12. This is typically the position in which the torque on the cartridge plunger 72 can reach the pre-defined threshold in order to disengage the cartridge plunger 72 from the sleeve 66.

Fig. 4f shows a sectional view taken along the section A:A of Fig. 4e. In this drawing like in Fig. 4e one can see that the abutment 94 is arranged at the same axial height as the second holding member 86 which in turn may be formed by the plurality of cams 86' within the sleeve 66.

Fig. 4g shows an enlarged view of part of the section of Fig. 4f. In this view both the plurality of cams 86' as well as the plurality of teeth 84' respectively forming the second and first holding members 86, 84 are visible. If a torque less than a pre-defined threshold is applied at the plunger then the teeth 84' mesh with the cams 86' to rotate the sleeve 66. If a torque exceeding the pre-defined threshold is applied, then the teeth 84' slip over the cams 86' so that the cartridge plunger 72 can move relative to the sleeve 66.

On activating the mixing and dispensing system 10 illustrated in Figs. 1a and 1b, the activation element 24 is moved from the storage position into the activated position, thereby the part 26, i.e. the activation plunger 26', of said activation element 24 is moved perpendicular to said longitudinal axis A and comes into contact with the top 32 of the ampoule 18 to open this and to release the second component M" stored therein. The second component M" is brought into contact with the first component M' in said cartridge 12. The first and second components M', M" of the two-component material M', M" are then mixed in the chamber 50 of the cartridge 12 with said mixer 14.

To mix the two-component material M', M", the mixer is moved via the mixing shaft 54 and handle 52 to and fro along the longitudinal axis A and by rotating the handle 52, the mixer is also rotated about the longitudinal axis A. The vanes 62 and the outer ring 64 bring about a thorough through mixing of the two-component material M', M", as the mixer is moved through the chamber 50 and through the two-component material M', M".

It should be noted that the handle 52 and/or the mixing shaft 54 may have a socket, such as the hexagonal socket 150, via which the mixer 14 and the mixing shaft 54 can be set into rotation using e.g. a power tool. In this way more uniform mixing results of the two-component material M', M" can possibly be achieved.

In this connection it should be noted that the vacuum applied via the vacuum port 22 can be applied either before or during opening of the ampoule 18. If the vacuum is applied before breaking of the ampoule 18, this has the advantage that movement of the second component M" stored in the ampoule 18 into the cartridge 12 can be assisted through the use of the vacuum as soon as the ampoule 18 is open as the vacuum so to say sucks the second component M" into the cartridge 12.

Moreover, due to the presence of the vacuum the amount of air and thus pores in the mixed two-component material M', M" is significantly reduced, so that less air is present in the mixture reducing the amount of fatigue cracks that may appear in the solidified two-component material M', M", leading to improved adhesion results between the two-component material M', M" and the surfaces (not shown) between which it is applied.

Following the mixing of the two-component material M', M" with the mixing and dispensing system 10 the handle 52 connected to the mixer 14 via the mixing shaft 54 is detached. For this purpose a user presses the buttons 60 present at the connector 38. By pressing the buttons 60 the elements 58 more flexible than the mixing shaft 54 present at the rod 36' allow the rod 36' to disengage from the mixing shaft 54 in the regions of the apertures 55 present in the mixing shaft 54, whereby the rod 36' can be removed from the mixing shaft 54 to detach the handle 52 from the mixing shaft 54 via the connector 38.

In order to dispense material from the mixing and dispensing system 10 the user has to remove the ampoule container 16 from the cartridge 12 via the first interface 20. Following which the user can arrange a dispensing outlet 44, such as the dispensing outlet 44 shown in Fig. 4a, at the cartridge 12 using the first interface 20.

Following this the user now has several options to press the mixed two-component material M', M" out of the chamber 50 of the cartridge 12. One option is to install an insert (not shown) at the second interface 42, via which a dispensing gun can be connected to the cartridge 12, for dispensing the two-component material M', M" from the cartridge 12.

The other option is to use the dispensing mechanism 40' discussed in relation to Figs. 4a to 4g. This is done by attaching the sleeve 66 at the second interface 42, by turning the cartridge plunger 72, such that the first thread 68 of the sleeve 66 is turned into the inner thread 142. During the connection of the sleeve 66 to the cartridge the force on the sleeve 66 is below a pre-defined threshold value such that the sleeve 66 is entrained by the first and second holding members 84, 86 and transmits the rotations of the cartridge plunger 72 onto the sleeve 66. Once the abutment 94 contacts the cartridge 12, the sleeve 66 is finally installed at the second interface 42. As the sleeve 66 reaches its final position it also releases the piston 34 from the storage position for dispensing said two-component material M', M" from said cartridge 12.

In this final position the sleeve 66 experiences a resistance at the cartridge which means that an increased force has to be applied at the cartridge plunger 72 in order to rotate this further. This means that a force above the pre-defined threshold value is applied at the cartridge plunger 72, such that the first holding members 84 slip past the second holding members 86 so that the second thread 74 present at the outer surface outer surface 76 of the plunger can interact with the inner thread 78 present at an inner surface 80 of the sleeve 66.

The cartridge plunger 72 then comes into contact with the piston 34 for dispensing said two-component material M', M" from said cartridge 12. Once the cartridge plunger 72 has slipped past the first and second holding members 84, 86 one rotates only the cartridge plunger 72 via the engagement part 82. The engagement part 82 can be rotated either by hand or using a power tool (not shown) connectable to the engagement part via the coupling point 152.

On moving the piston 34 via the cartridge plunger 72, the mixer 14 may at least at some point of the dispensing process rest on the piston 34 and be entrained via the piston 34.

The dispensing mechanism 40' is designed in a way that allows a fast forwarding at the beginning, i.e. while the sleeve 66 is attached to the cartridge 12 via the second interface 42, and once the torque above a pre-defined threshold is achieved changes over to the cartridge plunger 72 having a lower pitch which transmits higher forces when needed for dispensing the two-component material M', M" via the dispensing outlet 44.

In this connection it should be noted that a material of at least one of the cartridge 12, the ampoule container 16, the cap 126 of the first interface 20, the cap 106 and the stand 110 and of the piston 34 may be polypropylene (PP).

A material of at least one of the handle 52, the rotatable switch 30, the rod 36', and the threaded dispenser 40 may be one of PP and polyamide (PA).

The net 102 and of the membrane 130 may be formed of a thermoplastic elastomer (TPE).

Using the above mixing and dispensing system 10 a user, such as a medical professional, can minimize exposure to the two-component material M', M". If, for example PMMA is used as a Monomer then this Monomer can be disturbing for the respiratory tract. The use of the ampoule container described in the foregoing, the disconnectable handle and the dispensing mechanism 40' reduces the risk of the medical professional coming into contact with the monomer. Moreover, if the dispensing mechanism 40' is used one can use the mixing and dispensing system 10 independent of a multiple-use dispensing gun.

### List of reference numerals:

- 10: mixing and dispensing system
- 12: cartridge
- 14: mixer
- 16: ampoule container
- 18: ampoule
- 20: first interface
- 22: vacuum port
- 24: activation element
- 26, 26': part of 24, activation plunger
- 28: outer wall of 16
- 30, 30': rotatable switch, quarter turn coupling
- 32: top of 18
- 34: piston
- 36, 36': part of 14, rod
- 38, 38': connector, drive interface
- 40, 40': threaded dispenser
- 42: second interface
- 43: inlet of 44
- 44: dispensing outlet
- 45: outlet of 44
- 46: piston driving end
- 48: outlet end
- 50: chamber
- 52: handle
- 54: mixing shaft
- 55: aperture
- 56: piston passage
- 58: elements
- 60: button
- 62: vane
- 64: outer ring
- 66: sleeve
- 68: first thread
- 70: outer surface of 66
- 72: cartridge plunger
- 74: second thread
- 76: outer surface of 72
- 78: inner thread of 66
- 80: inner surface of 66
- 82: engagement part
- 84, 84': first holding member, tooth
- 86, 86': second holding member, cam
- 88: inner surface of 12
- 90: cartridge wall
- 92: passage
- 94: abutment
- 96: groove
- 98: vacuum line
- 100: passage
- 102: net
- 104: end
- 106: cap
- 108: end
- 110: stand
- 112: groove for 54
- 114: groove for 52
- 116: support of 110
- 118: end of 110
- 120: outer surface of 12
- 122: pin
- 124: slot
- 126: cap
- 128: aperture
- 130: membrane
- 132: inner thread of 30
- 134: outer thread
- 136: projection
- 138: outer surface of 16
- 140: neck of 18
- 142: inner thread of 12
- 144: bar
- 146: end
- 148: hexagonal fitting
- 150: hexagonal socket
- 152: coupling point

- A: longitudinal axis
- M', M": first component of material, second component of material
- R: axis of rotation of 30

## Claims

1. A dispensing mechanism (40') for dispensing material (M', M") from a cartridge (12), the dispensing mechanism (40') comprising:
- a sleeve (66) having a first thread (68) arranged at an outer surface (70) thereof for connecting the sleeve (66) to an end of the cartridge (12);
- a cartridge plunger (72) that can be arranged partly within said sleeve (66), the cartridge plunger (72) being moveable with and relative to said sleeve (66), and the cartridge plunger (72) having a second thread (74) arranged at an outer surface (76) thereof that can interact with an inner thread (78) present at an inner surface (80) of the sleeve (66); and
- an engagement part (82) at said cartridge plunger (72) for rotating at least one of the cartridge plunger (72) and said sleeve (66);
wherein first and second holding members (84, 86) are present between the cartridge plunger (72) and said sleeve (66), with the first and second holding members (84, 86) being configured to hold the cartridge plunger (72) at the sleeve (66) when a torque below a pre-defined threshold is applied at the cartridge plunger (72).

2. A dispensing mechanism (40') according to claim 1,
wherein the first and second holding members (84, 86) are configured to release the cartridge plunger (72) from the sleeve (66) once a torque above a pre-defined threshold is applied at the cartridge plunger (72).

3. A dispensing mechanism (40') according to claim 1 or claim 2,
wherein the first holding member (84) is formed at an outer surface (76) of the cartridge plunger (72) and comprises a plurality of teeth (84').

4. A dispensing mechanism (40') according to one of the preceding claims, wherein the second holding member (86) is formed at the inner surface (80) of the sleeve (66) and comprises a plurality of cams (86').

5. A dispensing mechanism (40') according to claim 3 and claim 4,
wherein the plurality of teeth (84') and the plurality of cams (86') are formed complementary to one another.

6. A dispensing mechanism (40') according to claim 3 and claim 4 or claim 5, wherein the plurality of teeth (84') are configured to slip over the plurality of cams (86') when a torque applied at said cartridge plunger (72) exceeds the pre-defined threshold.

7. A dispensing mechanism (40') according to one of the preceding claims, wherein the first and second threads (68, 74) have different pitches.

8. A dispensing mechanism (40') according to one of the preceding claims, wherein the second thread (74) is finer than the first thread (68), i.e. the second thread (74) has a lower thread pitch than the first thread (68).

9. A dispensing mechanism (40') according to one of the preceding claims, further comprising a cartridge (12), optionally filed with material (M', M"), wherein the first thread (68) is configured to engage an inner surface (88) of a cartridge wall (90) of the cartridge (12).

10. A dispensing mechanism (40') according to claim 9,
wherein the sleeve (66) is configured to release a piston (34) present in said cartridge (12) from a storage position of said piston (34).

11. A dispensing mechanism (40') according to claim 10,
wherein the cartridge plunger (72) is configured to move the piston (34) present in said cartridge (12) to and fro along a longitudinal axis of said cartridge (12) once the sleeve (66) has released the piston (34) from the storage position of said piston.

12. A dispensing mechanism (40') according to one of the preceding claims, wherein the cartridge plunger (72) comprises a passage (92) configured for receiving a mixing shaft (54) of a mixer (14) arranged within said cartridge (12).

13. A dispensing mechanism (40') according to one of the preceding claims, wherein the sleeve (66) comprises an abutment (94) at the outer surface (70) thereof for abutting against an end of the cartridge (12).

14. A dispensing mechanism (40') according to claim 13,
wherein the abutment (94) is arranged at the same axial height of the sleeve (66) as the second holding member (86).

15. A method of dispensing material using a dispensing mechanism (40'), in particular according to one of the preceding claims, the dispensing mechanism (40') comprising:
- a sleeve (66) having a first thread (68) arranged at an outer surface (70) thereof for connecting the sleeve (66) to an end of the cartridge (12);
- a cartridge plunger (72) that can be arranged partly within said sleeve (66), the cartridge plunger (72) being moveable with and relative to said sleeve (66), and the cartridge plunger (72) having a second thread (74) arranged at an outer surface (76) thereof that can interact with an inner thread present at an inner surface (80) of the sleeve (66); and
wherein first and second holding members (84, 86) are present between the cartridge plunger (72) and said sleeve (66), with the first and second holding members (84, 86) being configured to hold the cartridge plunger (72) at the sleeve (66) when a torque below a pre-defined threshold is applied at the cartridge plunger (72), the method comprising the steps of:
- rotating an engagement part (82) of said cartridge plunger (72) while connecting the sleeve (66) to the cartridge (12); and
- further rotating said engagement part (82) to rotate only said cartridge plunger (72) once a torque has been applied at said cartridge plunger (72) that is above said pre-defined threshold.
